# EUROPEAN PATENT SPECIFICATION

(11) **EP 2 595 485 B1**
(45) Date of publication and mention of the grant of the patent: **16.03.2022**
(21) Application number: 11810350.6
(22) Date of filing: 20.07.2011
(51) Int. Cl.: A61K 31/454, A61P 25/18, A61P 25/24

(54) **METHODS OF USE CYCLIC AMIDE DERIVATIVES TO TREAT SIGMA RECEPTOR-MEDIATED DISORDERS**
VERFAHREN ZUR VERWENDUNG ZYKLISCHER AMIDDERIVATE ZUR BEHANDLUNG SIGMA-REZEPTOR-VERMITTELTER STÖRUNGEN
PROCÉDÉS D'UTILISATION DE DÉRIVÉS D'AMIDES CYCLIQUES DESTINÉS À TRAITER DES TROUBLES INDUITS PAR LE RÉCEPTEUR SIGMA

(30) Priority: 20.07.2010 US 366080 P
(43) Date of publication of application: 29.05.2013
(73) Proprietor: Minerva Neurosciences, Inc., Waltham, MA 02451 (US)
(72) Inventor: LUTHRINGER, Remy, Henri, 1204 Geneve (CH); PELLEGRINI, Lorenzo, Newtown, PA 18940 (US); KARABELAS, Argeris, N., Portsmouth, NH 03801 (US)
(74) Representative: Cooley (UK) LLP
(86) International application number: PCT/US2011/044698
(87) International publication number: WO 2012/012543

(56) References cited:
- EP-A1- 1 260 512
- US-B2- 7 166 617
- BENTHAM SCIENCE PUBLISHER BENTHAM SCIENCE PUBLISHER: "Sigma Receptor Ligands: Possible Application as Therapeutic Drugs and as Radiopharmaceuticals", CURRENT PHARMACEUTICAL DESIGN, vol. 12, no. 30, 1 October 2006 (2006-10-01), pages 3857-3876, XP055107718, ISSN: 1381-6128, DOI: 10.2174/138161206778559614
- anonymous: "View of NCT00861796 on 2009_03_12", ClinicalTrials.gov archive , 12 March 2009 (2009-03-12), XP007922577, Retrieved from the Internet: URL:http://clinicaltrials.gov/archive/NCT0 0861796/2009_03_12 [retrieved on 2014-03-13]
- MARDER ET AL.: 'Aripiprazole in the treatment of schizophrenia: safety and tolerability in short-term, placebo-controlled trials.' SCHIZOPHRENIA RESEARCH vol. 61, no. IS.2-3, 01 June 2003, pages 123 - 136, XP055105227
- KAY S R ET AL: "THE POSITIVE AND NEGATIVE SYNDROME SCALE (PANSS) FOR SCHIZOPHRENIA", SCHIZOPHRENIA BULL, OXFORD UNIVERSITY PRESS, vol. 13, no. 2, 1 January 1987 (1987-01-01), pages 261-276, XP009020866, ISSN: 0586-7614

## Description

### BACKGROUND

Sigma receptors are found throughout the body, and play a number of different roles as pharmacological targets. For example, the sigma receptor/binding sites of the brain are important targets for the development of antipsychotic drugs that are free from the side affects of traditional antipsychotic drugs having antagonistic activity on the dopamine D2 receptor (see, E.g., J. M. Walker et al., Pharmacological Reviews, 42:355-402, 1990).

The sigma receptor exists in two subtypes, sigma 1 and sigma 2. The sigma 1 binding site was characterized to have high affinity for haloperidol, di-o-tolylguanidine (DTG) and (+)-benzomorphanes such as (+)-pentazocine. The sigma 2 binding site is characterized to have high affinity for haloperidol and DTG, but have low affinity for (+)-benzomorphane.

Sigma 1 ligands may act on the gastrointestinal tract. The sigma 1 site may mediate suppression to muscarine-like acetylcholine receptor/phosphoinositide response by the sigma ligands. The sigma 1 binding site is present not only in brains, but on spleen cells (Y. Lin et al., J. Neuroimmunol., 58:143-154, 1995), and such sigma ligands may suppress the immune system (H. H. Garza et al., J. Immunol., 151:4672-4680, 1993).

The sigma 2 binding site is abundant in livers (A. E. Bruce et al., Neurosci. Abstr., 16:370, 1990), kidneys (W. D. Bowen et al., Soc. Neurosci. Abstr., 18:456), and heart (M. Dumont and S. Lemaire, Eur. J. Pharmacol., 209:245, 248, 1991). The sigma 2 binding site in brain exists in the hypothalamus, cerebellum, pons medulla and medulla oblongata. In hippocampus, frontal lobe and occipital lobe in rat brains, it exists more abundantly than the sigma 1 binding site. In hippocampal synaptosomes of guinea pig, there is a sigma 2 binding site that is selectively labeled with [³H] BIMU (D. W. Bonhaus et al., J. Pharmacol. Exp. Ther., 267:961-970, 1993). The relationship between the sigma 2 binding site and cortex as well as limbic system supports the usefulness of compounds used for treatment of mental diseases (D. C. Mash and C. P. Zabetian, Synapse, 12:195-205, 1992). It has been believed that the sigma 2 binding site is involved in motility functions, especially dystonia, however, no evidence demonstrating such an action has been found in primate models of functional disorders of extrapyramidal tract (L. T. Meltzer et al., Neuropharmacology, 31:961-967, 1992).

Agonists of sigma-2 receptors can induce changes in cell morphology and apoptosis in various cell types (W.D. Bowen, 74:211-218, 2000). Sigma-2 receptor activation produces both transient and sustained increases in [Ca++]i, derived from different intracellular stores. The changes in [Ca++]i and also cytotoxic effects are mediated by intracellular sigma-2 receptors. Additionally, it has been shown that sigma-2 agonists induced apoptosis in drug-resistant cancer cells, enhanced the potency of DNA damaging agents, and down-regulated expression of p-glycoprotein mRNA (implicating some sigma-2 receptor agonists as useful in treatment of drug-resistant cancers). Prior work has suggested that sigma-2 antagonists might prevent the irreversible motor side effects of typical neuroleptics, and that some sigma-2 receptors may serve as a novel signaling pathway to apoptosis, involved in regulation of cell proliferation and/or viability.

Haloperidol, a clinically effective dopaminergic antipsychotic agent, shows high affinity for both sigma subtypes 1 and 2. However, a reduced metabolite of haloperidol that acts on the central nervous system has higher affinity and selectivity for the sigma 2 receptor than dopamine D2, as compared to haloperidol (J. C. Jaen.et al., J. Med. Chem., 36:3929-3936, 1993).

U.S. Patent 7,166,617 discloses cyclic amide derivatives having high affinity for the sigma 2 binding site. Certain compounds disclosed in this patent also have high affinity for the sigma ligand binding site and low inhibition constant Kᵢ for sigma 1 and/or sigma 2, as well as selective binding profiles completely different from those of conventional known compounds. Such compounds may be useful for treatment of diseases that can be therapeutically and/or preventively treated by the nerve control function of the sigma ligands. However, the properties and characteristics of specific derivatives were not disclosed in U.S. Patent 7,166,617.

Marder et al. disclose, in Schizophrenia Research, 61:123-136, 2003, "Aripiprazole in the treatment of schizophrenia: safety and tolerability in short-term, placebo-controlled trials."

### DETAILED DESCRIPTION OF THE INVENTION

The present invention provides a compound of the formula (II) or a pharmaceutically acceptable salt, hydrate, or solvate thereof, 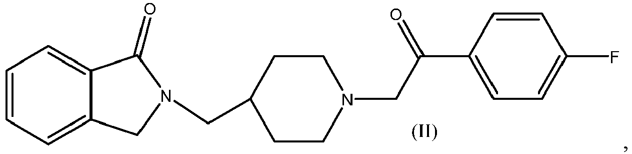 for use in the treatment of schizoaffective disorder, or in the treatment or alleviation of at least one symptom of schizoaffective disorder in a subject suffering from schizoaffective disorder.

In some embodiments, the compound is a hydrochloride salt.

In some embodiments, the symptom is task completion issues.

In some embodiments, the symptom is dysthymia.

In some embodiments, at least two symptoms are treated.

### Definitions

"Modulate", as this term is used herein, includes, but is not limited to, stabilizing, promoting, increasing, inhibiting or disrupting protein-protein interactions (e.g., homophilic and/or heterophilic). As used herein, "modulate" also refers to affecting the interaction between non-protein molecules and receptor molecules.

As used herein, the term "schizophrenia" covers the full spectrum of schizophrenic disorders known to the skilled person. These include, but are not limited to, the following: catatonic, disorganized, paranoid, residual and undifferentiated schizophrenia; schizophreniform disorder and schizoaffective disorder.

The term "receptor", as used herein, means a membrane-binding type receptor, as well as other binding sites. For example, the existence of at least two sigma receptor subtypes is known, i.e., sigma 1 and sigma 2, and classification of sigma binding sites has been proposed (R. Quirion et al., TiPS, 13:85-86, 1992).

The term "subject" refers to any animal, including mammals, such as, but not limited to, humans, mice, rats, other rodents, rabbits, dogs, cats, pigs, cattle, sheep, horses, or primates.

The term "treating" (and corresponding terms "treat" and "treatment") includes palliative, restorative, and preventative ("prophylactic") treating of a subject. The term "palliative treating" refers to treatment that eases or reduces the effect or intensity of a condition in a subject without curing the condition. The term "preventative treating" (and the corresponding term "prophylactic treating") refers to treatment that prevents the occurrence of a condition in a subject. The term "restorative treating" ("curative") refers to treatment that halts the progression of, reduces the pathologic manifestations of, or entirely eliminates a condition in a subject. Treating can be done with a therapeutically effective amount of compound, salt or composition that elicits the biological or medicinal response of a tissue, system or subject that is being sought by an individual such as a researcher, doctor, veterinarian, or clinician.

"PANSS" refers to the Positive and Negative Syndrome Scale.

"BACS" refers to the Brief Assessment of Cognition in Schizophrenia test.

"HAMD" refers to the Hamilton Depression Rating Scale.

"HAMA" refers to the Hamilton Anxiety Scale.

"ADAS COG" refers to the Alzheimer's Disease Assessment Scale - cognitive subscale and test.

"MADRS" refers to the Montgomery-Asberg Depression Rating Scale.

"PSQI" refers to the Pittsburgh Sleep Quality Index.

The invention provides a compound of the formula (II) for use in the treatment of schizoaffective disorder, which is a sigma-2 receptor related disorder/condition, or in the treatment or alleviation of at least one symptom of schizoaffective disorder in a subject suffering from schizoaffective disorder. It will be understood by the skilled artisan that any condition which is mediated by or relies upon the sigma-2 receptor may be affected by a compound that modulates the sigma receptor. Other sigma-2 receptor related conditions (not encompassed by the claims) include behavioral and cognitive components of Alzheimer's Disease, Lewey Body dementia, bipolar disorders, attention deficit disorder, attention deficit hyperactivity disorder, acquired immune deficiency syndrome-related dementia, depression, hypomania, and addiction.

In an example which is not encompassed by the claims, compounds of formula I have been shown to be useful to treat schizophrenia

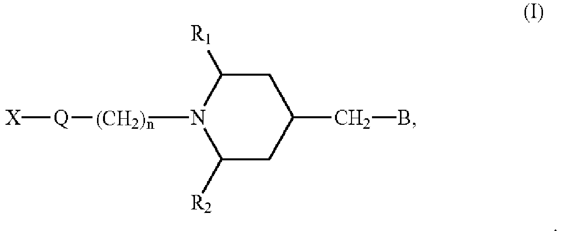

In another example which is not encompassed by the claims, compounds of formula I have been shown to be useful to treat one or more symptoms of schizophrenia. Such symptoms may be negative symptoms, positive symptoms, or general symptoms.

The invention provides a compound of the formula (II) for use in the treatment of schizoaffective disorder, or in the treatment or alleviation of at least one symptom of schizoaffective disorder in a subject suffering from schizoaffective disorder. In one aspect, the symptom is task completion issues. In another aspect, the symptom is dysthymia.

The compound of formula (II) is also referred to herein as CYR-101: 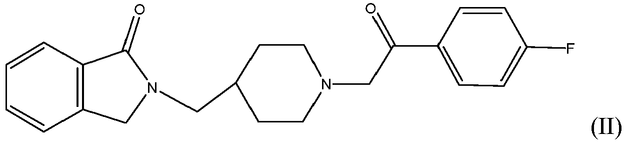 2-[[1-[2-(4-Fluorophenyl)-2-oxoethyl]piperidin-4-yl]methyl]isoindolin-1-one.

Also described herein (but not encompassed by the claims) is the compound set forth in formula (III):

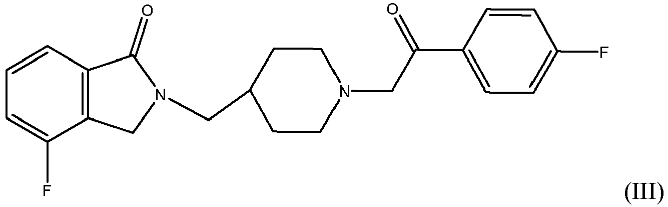

The compound of formula III may have properties and activity similar to a compound of formula II.

The compounds disclosed herein have a receptor binding profile demonstrating preferential binding for sigma 2 receptors, 5-HT_{2A} receptors, and α₁ adrenergic receptors. However, it will be understood that certain such compounds may not have a preferential binding for the same panel of receptors, and in some instances, may demonstrate preferential binding for one or more different receptors, including fewer than all of the sigma 2, 5-HT_{2A}, and α₁ adrenergic receptors. For example, compounds disclosed herein may have little or no affinity for dopaminergic, muscarinic, cholinergic or histaminergic receptors, and may have varying affinities for any combinations of those receptors. In one embodiment, a compound of formula II has little or no affinity for dopaminergic, muscarinic, cholinergic or histaminergic receptors.

In the compound of the formula (I), above (which is not encompassed by the claims), X represents an alkyl group, a cycloalkyl-substituted alkyl group, an aryl-substituted alkyl group, an aryl-substituted alkenyl group, an aryl-substituted alkynyl group, a monocyclic or polycyclic cycloalkyl group which may be substituted with an alkyl group, an aryl group, a heterocyclic group, or a substituted or unsubstituted amino group; Q represents a group represented by --CO--, --O--, --S--, --CH(OR₇)--, --C(=CH₂)- - or --C(=NR₈)-- wherein R₇ represents a hydrogen atom, an alkyl group, a hydroxyalkyl group, or an acyl group, and R₈ represents a hydroxyl group, an alkoxyl group, an aralkyloxy group, an acyloxy group, an acylamino group, or an alkoxycarbonyl amino group; n represents an integer of from 0 to 5; R₁ and R₂ each independently represent a hydrogen atom or an alkyl group.

B represents the following groups:

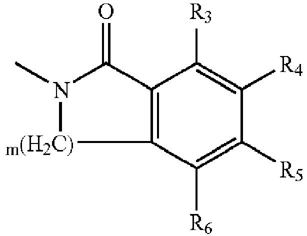

wherein R₃, R₄, R₅ and R₆ each independently represent a substituent selected from the group consisting of a hydrogen atom, a halogen atom, a nitro group, an alkyl group, a halogenated alkyl group, a hydroxyl group, an alkoxyl group, a halogenated alkoxyl group, and a cyano group; and m represents 1 or 2.

### Dosage Forms and Amounts

For therapeutic administration according to the present invention, the compound of formula (II) may be employed in the form of its free base, but is preferably used in the form of a pharmaceutically acceptable salt, typically the hydrochloride salt.

Alternative salts of the compound of formula (II) with pharmaceutically acceptable acids may also be utilized in therapeutic administration, for example salts derived from the functional free base and acids including, but not limited to, palmitic acid, hydrobromic acid, phosphoric acid, acetic acid, fumaric acid, maleic acid, salicylic acid, citric acid, oxalic acid, lactic acid, malic acid, methanesulphonic acid and p-toluene sulphonic acid.

All solvates and all alternative physical forms of a compound of formula (II) or its pharmaceutically acceptable derivatives as described herein, including but not limited to alternative crystalline forms, amorphous forms and polymorphs, are also within the scope of this invention, and all references to a compound of formula (II) herein include all pharmaceutically acceptable salts, and all solvates and alternative physical forms thereof.

For therapeutic administration, the compound of formula II may be administered in pure form, but will preferably be formulated into any suitable pharmaceutically acceptable and effective composition which provides effective levels of the active ingredient in the body.

Preferred forms include, but are not limited to, depot formulations (E.g., crystalline, emulsion), depot formulations suitable for intra-muscular or sub-dermal injection, controlled release forms, including controlled release tablets, transdermal systems (E.g., patch), buccal forms (E.g., film, tablet), effervescent tablets, and sub-dermal trochy. In an embodiment, a depot formulation comprises a palmitate salt of a compound of formula (II).

Suitable doses of the compound or pharmaceutically acceptable salt thereof may be between 10 µg - 200 mg, 15 µg - 190 mg, 25 µg - 180 mg, 50 µg - 170 mg, 75 µg - 160 mg, 100 µg - 150 mg, 250 µg - 140 mg, 400 µg - 130 mg, between 500 µg - 128 mg, 600 µg - 100 mg, 750 µg - 75 mg, 900 µg - 50 mg, or at a dose between 1 mg - 64 mg. A suitable dose may be <200 mg, <150 mg, <100 mg, <50 mg, <40 mg, <30 mg, <20 mg, <10 mg, <9 mg, <8 mg, <7 mg, <6 mg, <5 mg, <4 mg, <3 mg, <2 mg, <1 mg, <0.5 mg, <0.25 mg, <0.1 mg, <0.05 mg, or <0.01 mg.

### Co-Administration of Compounds

The compound of formula (II) or a pharmaceutically acceptable salt thereof may be administered independently of any other medication. Alternatively, the compound of formula (II) or a pharmaceutically acceptable salt thereof may be administered in conjunction with one or more other medications.

The skilled artisan will understand that the at least one additional compound will be selected based on the disorder or condition to be treated. By way of a non-limiting example, treatment of anxiety may comprise treatment of a patient in need thereof with a compound of formula II in conjunction with at least one anti-anxiolytic compound. Similarly, the skilled artisan will understand how to identify a therapeutically effective dose of the additional compound, based on the patient, the condition, the compound, and the information known regarding the properties and activity of the additional compound.

By way of another non-limiting example, the compound set forth in formula II, or a pharmaceutically acceptable salt thereof, may advantageously be administered in combination with at least one neuroleptic agent (E.g., a typical or an atypical antipsychotic agent). The present invention may also provide advantages in treatment of patients who fail to respond adequately or who are resistant to other known treatments.

The compound of formula II may be administered to a patient already undergoing treatment with at least one neuroleptic agent (E.g., a typical or an atypical antipsychotic agent).

### EXPERIMENTAL EXAMPLES

### Example 1: Clinical Study of CYR-101

A study was conducted using the compound of formula II, to examine the efficacy on schizophrenia and treatment of symptoms of schizophrenia. The study was a multi-center, inpatient and ambulatory, phase 2, double-blind, randomized, placebo-controlled proof of concept study of the compound of formula II in patients with DSM-IV schizophrenia. The study used 21 centers across three different countries.

The study was designed to test the therapeutic efficacy of the compound of formula II on all dimensions of schizophrenic disease (E.g., positive, negative, and general symptoms, cognition, sleep, mood and anxiety). The study also examined the safety of the administered doses of the compound of formula II (also referred to herein as CYR-101), including heart repolarization (i.e., QT interval), weight change, adverse events, prolactin, and extrapyramidal symptoms).

The study was conducted for a time period of three months. This time period was sufficient to allow for the compound to demonstrate full therapeutic potential, particularly with respect to cognitive parameters.

The objectives of the study included the following:

| |
|---|
| 1. Evaluate the efficacy versus placebo of CYR-101 on global PANSS score and sub-scores after one month (28 days +/- 2 days) of treatment |
| 2. Test whether the administered dose of CYR-101 will demonstrate significantly greater efficacy as assessed by PANSS total score and sub-scores after three months (84 days +/-2 days) of treatment |
| 3. Evaluate the efficacy versus placebo of CYR-101 as assessed by the CGI-S after one and three months of treatment |
| 4. Evaluate subjective efficacy in patients of CYR-101 versus placebo as assessed |

| |
|---|
| by the Drug Attitude Inventory-10 (DAI-10) after one and three months of treatment |
| 5. Evaluate subjective sleep quality as assessed by Pittsburgh Sleep Quality Index (PSQI) after three month of treatment |
| 6. Explore the efficacy versus placebo of CYR-101 on cognitive function as measured by BACS (Brief Assessment of Cognition in Schizophrenia) scale after one and three months of treatment |
| 7. Evaluate the efficacy versus placebo of CYR-101 in depressive symptoms as measured by the Montgomery-Asberg Depression Rating Scale (MADRS) total score after one month of treatment |
| 8. Evaluate the efficacy versus placebo of CYR-101 in anxiety as measured by the Hamilton Anxiety Scale (HAMA) total score after one month of treatment |
| 9. Assess cardio-vascular safety (particularly ventricular repolarisation as assessed by QT/QTc interval measurements) of CYR-101 compared with placebo |
| 10. Assess the global safety and tolerability of CYR-101 compared with placebo |
| 11. Determine the pharmacokinetics of CYR-101 in schizophrenic patients |

In one aspect of the study, the dosage of compound was titrated up to 32 mg b.i.d.

The resulting data was analyzed one of three ways: 1.) Safety set; 2.) Full analysis set, with each patient having at least one PANSS evaluation after treatment initiation included in the efficacy analysis. The LOCF method is used; and 3.) Per protocol set, where for certain analyses, all patients having completed three months of treatment are included. ANCOVA followed by a contrast analysis at each time point were applied and in some cases, a non-parametric Wilcoxon test was used.

The results showed no significant difference between CYR-101 and placebo groups with respect to the emergence or worsening of extrapyramidal symptoms. There were three statistically significant adverse events (SAE), two of which were in the placebo group. The one SAE in the active treatment group was unlikely related to CYR-101 based on the patient history.

Improvement in negative symptoms was observed immediately, and continued through the course of treatment. This effect of the compound was surprising. Positive symptoms did not improve until after the first four weeks of treatment. Moreover, improvement in both positive and negative symptoms continued for more than twelve weeks. This is also surprising, as other antipsychotics typically only show improvement for six weeks.

Further, it is noted that CYR-101 has a positive effect on cognition in schizophrenic patients. Cognition was shown to improve quickly upon beginning treatment of patients with CYR-101. Cognitive performances assessed by the mean of the BACS show on the FAS, no differences between the placebo group and the CYR-101 group, except for the Token motor task. On the PPC at D84, descriptive data show a slight difference in favour of CYR-101 group in comparison to the placebo group for the Token motor task, list learning task and for verbal fluency, as well as for processing speed. These differences were not statistically significant. However, in comparison, it should be noted that most other antipsychotic treatments have a marked negative effect on cognition.

An increase in the QT interval was observed after CYR-101 was administered at doses up to 32 mg b.i.d. However, the observed increase remained stable over time and did not cross clinically acceptable limits (E.g., 10-15 milliseconds or less).

In summary, CYR-101 induced surprising and unexpected immediate and sustained effects on negative symptoms and some cognitive functions disturbed in schizophrenic patients. CYR-101 has also some effects on positive symptoms but there is a need of a longer period of treatment to start to see a differentiation from placebo. All the above mentioned effects are accompanied by some improvements of mood, anxiety and sleep, making CYR-101 a desirable basis for therapy to treat schizophrenia and symptoms of schizophrenia with a minimum of side effects and an advantageous, immediate, and beneficial effect on negative symptoms and cognition.

### Example 2: Receptor Binding Profile of CYR-101

U.S. Patent 7,166,617 illustrates the preferential binding of CYR-101 to the sigma 2 receptor site. The test compound of Example 1 of U.S. Patent 7,166,617 is CYR-101. As illustrated in Table 3 in U.S. Patent 7,166,617, CYR-101 has an affinity of 13 nM for the sigma 2 receptor. This data illustrates that CYR-101 demonstrates sigma 2-selective receptor binding. Furthermore, it is known that CYR-101 is a dual 5-HT2A /sigma 2 antagonist and is devoid of dopamine binding properties.

## Claims

1. A compound of the formula (II) or a pharmaceutically acceptable salt, hydrate, or solvate thereof, 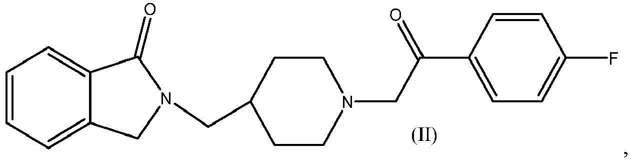 for use in the treatment of schizoaffective disorder, or in the treatment or alleviation of at least one symptom of schizoaffective disorder in a subject suffering from schizoaffective disorder.

2. The compound, salt, solvate or hydrate for use according to claim 1, wherein the compound is a hydrochloride salt.

3. The compound, salt, solvate or hydrate for use according to claim 1 or 2, wherein the symptom is task completion issues.

4. The compound, salt, solvate or hydrate for use according to claim 1 or 2, wherein the symptom is dysthymia.

5. The compound, salt, solvate or hydrate for use according to any one of claims 1-4, wherein at least two symptoms are treated.

## Patentansprüche

1. Verbindung der Formel (II) oder ein pharmazeutisch unbedenkliches Salz, Hydrat oder Solvat davon, 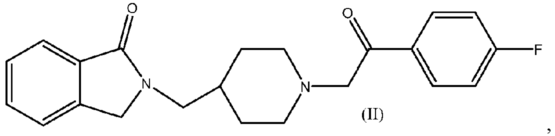 zur Verwendung bei der Behandlung von schizoaffektiver Störung oder bei der Behandlung oder Linderung von mindestens einem Symptom von schizoaffektiver Störung bei einem Individuum, das an schizoaffektiver Störung leidet.

2. Verbindung, Salz, Solvat oder Hydrat zur Verwendung nach Anspruch 1, wobei es sich bei der Verbindung um ein Hydrochloridsalz handelt.

3. Verbindung, Salz, Solvat oder Hydrat zur Verwendung nach Anspruch 1 oder 2, wobei es sich bei dem Symptom um Aufgabenerfüllungsprobleme handelt.

4. Verbindung, Salz, Solvat oder Hydrat zur Verwendung nach Anspruch 1 oder 2, wobei es sich bei dem Symptom um Dysthymie handelt.

5. Verbindung, Salz, Solvat oder Hydrat zur Verwendung nach einem der Ansprüche 1-4, wobei mindestens zwei Symptome behandelt werden.

## Revendications

1. Composé de la formule (II) ou sel, hydrate ou solvate pharmaceutique acceptable correspondant, 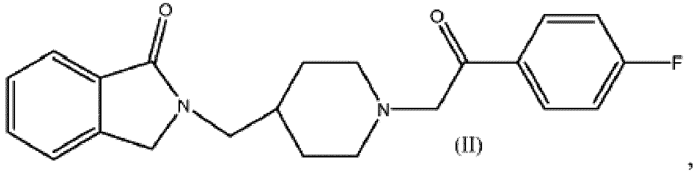 pour une utilisation dans le traitement d'un trouble schizoaffectif, ou dans le traitement ou l'atténuation d'au moins un symptôme de trouble schizoaffectif chez un sujet souffrant d'un trouble schizoaffectif.

2. Composé, sel, solvate ou hydrate pour une utilisation selon la revendication 1, le composé étant un sel de chlorhydrate.

3. Composé, sel, solvate ou hydrate pour une utilisation selon la revendication 1 ou 2, le symptôme étant des problèmes d'accomplissement de tâches.

4. Composé, sel, solvate ou hydrate pour une utilisation selon la revendication 1 ou 2, le symptôme étant une dysthymie.

5. Composé, sel, solvate ou hydrate pour une utilisation selon l'une quelconque des revendications 1 à 4, au moins deux symptômes étant traités.
